# DEMANDE DE BREVET EUROPEEN

(11) **EP 4 799 604 A1**
(43) Date de publication de la demande: **02.09.2026**
(21) Numéro de dépôt: 26160873.1
(22) Date de dépôt: 26.02.2026
(51) Int. Cl.: A61K 8/41, A61K 8/92, A61Q 5/10

(54) **COMPOSITION COSMETIQUE DE COLORATION CAPILLAIRE PERMANENTE A USAGE TOPIQUE, PROCEDE ET KIT DE COLORATION AFFERENTS**

(30) Priorité: 28.02.2025 FR 2502073
(71) Demandeur: CENTRE DE TRAITEMENT ET DE REGENERATION DU CHEVEU JF LAZARTIGUE, 75008 Paris (FR)
(72) Inventeur: MENY, Camille, 75008 PARIS (FR); TAMELGHAGHET, Myriam, 75008 PARIS (FR)
(74) Mandataire: Cabinet Didier Martin

(57) **Abrégé**

L'invention concerne une composition cosmétique de coloration capillaire permanente à usage topique, qui est auto-oxydante et prête à l'emploi sans mélange avec un oxydant et qui comprend : au moins un précurseur de colorant oxydatif et un agent alcalin pour favoriser la pénétration dudit précurseur de colorant oxydatif au sein de cheveux à colorer, ledit précurseur de colorant oxydatif étant conçu pour être oxydé uniquement par exposition à l'air ambiant pendant au moins une durée prédéterminée pour former, au sein des cheveux à colorer, un colorant de couleur prédéterminée, ladite composition cosmétique incluant en outre au moins une huile végétale, qui inclut de l'huile de graines de *Camellia oleifera* et qui forme un agent filmogène pour favoriser au moins temporairement la rétention dudit colorant au sein des cheveux.

## Description

La présente invention se rapporte au domaine technique général des produits cosmétiques à usage topique, utilisables notamment sur les cheveux, et pouvant notamment se présenter sous forme fluide, liquide, de crème, de gelée, ou de mousse.

La présente invention concerne plus précisément une composition cosmétique de coloration capillaire permanente à usage topique.

Les kits de colorations capillaires sont bien connus de longue date. Ils incluent généralement une crème de coloration, destinée à être appliquée sur la chevelure. Cette crème de coloration contient des pigments utilisés pour colorer les cheveux. Les kits de colorations capillaires connus comprennent également un produit oxydant (révélateur) se présentant sous la forme d'un liquide qui contient du peroxyde d'hydrogène. Le révélateur est destiné à être mélangé avec la crème de coloration pour oxyder les pigments présents dans la crème et assurer ainsi l'effet de coloration. Plus précisément, lors de l'oxydation les pigments contenus dans la crème colorante réagissent pour former des macromolécules colorées qui se fixent dans la fibre capillaire.

Ces kits de colorations connus donnent généralement satisfaction. Ils permettent d'obtenir en particulier une excellente rémanence de la coloration, qui reste visible et intense pendant une longue durée, malgré les lavages, l'exposition au soleil et autres agressions extérieures. Toutefois, ils n'en présentent pas moins des inconvénients sérieux.

En effet, ces kits sont basés sur un mélange d'au moins deux composants, savoir la crème colorante d'une part et le révélateur d'autre part, ce qui est de nature à complexifier l'utilisation et à en accroître les risques, notamment en raison de la présence de peroxyde d'hydrogène.

De son côté, CN 114146029 A divulgue une teinture capillaire qui, dans sa formulation générale, contient, en poids, 2 à 6 % de produits de fermentation d'aspergillus, 1 à 3 % de 5,6-dihydroxyindole, 4 à 8 % d'alcool en C16-C18, 12 à 16 % d'huile d'olive, 0,5 à 2 % d'huile d'olive PEG-7, 2 à 8 % de propylène glycol, 1 à 6 % de cystéine, 0,1 à 0,2 % de sulfite de sodium, 0,1 à 0,5 % d'éther éthylique d'acide ascorbique, 0,2 à 2 % de polydiméthylsiloxane, 0,05 à 0,1 % de chlorure d'hydroxypropyltriméthylammonium de gomme de guar, 4 à 10 % d'éthanolamine, et, pour le reste, de l'eau. Après application de cette teinture sur les cheveux, le 5,6-dihydroxyindole s'oxyde uniquement par exposition à l'air ambiant pendant une durée d'au moins 20 minutes. Les produits de fermentation d'aspergillus sont utilisés pour limiter le taux d'oxydation du 5,6-dihydroxyindole à la surface des cheveux, permettant ainsi au 5,6-dihydroxyindole de pénétrer en plus grande quantité dans les écailles des cuticules capillaires. L'effet de coloration est principalement obtenu par le 5,6-dihydroxyindole et par les produits de fermentation d'aspergillus.

Les objets assignés à l'invention visent à remédier aux différents inconvénients exposés précédemment, et à proposer de nouveaux composition, procédé et kit de coloration capillaire qui sont extrêmement faciles, sûrs et rapides à mettre en œuvre, sans nécessiter de mélange, tout en permettant d'obtenir une couleur intense qui présente une excellente rémanence.

Un autre objet assigné à l'invention vise à proposer de nouveaux composition, procédé et kit de coloration capillaire qui permettent de colorer efficacement et durablement les cheveux tout en préservant la santé et l'intégrité de ces derniers.

Un autre objet de l'invention vise à proposer de nouveaux composition, procédé et kit de coloration capillaire qui reposent sur une formulation présentant un indice de naturalité élevé.

Un autre objet de l'invention vise à proposer de nouveaux composition, procédé et kit de coloration capillaire permettant une application sur les cheveux à colorer particulièrement facile, rapide et couvrante.

Un autre objet de l'invention vise à proposer de nouveaux composition, procédé et kit de coloration capillaire permettant une pénétration efficace de la coloration dans les fibres capillaires, pour assurer une coloration éclatante, durable et uniforme.

Un autre objet de l'invention vise à proposer de nouveaux composition, procédé et kit de coloration capillaire qui permettent d'optimiser la couverture des cheveux blancs.

Un autre objet de l'invention vise à proposer de nouveaux composition, procédé et kit de coloration capillaire permettant d'obtenir des cheveux particulièrement brillants, souples et doux.

Les objets assignés à l'invention sont atteints à l'aide d'une composition cosmétique de coloration capillaire permanente à usage topique, ladite composition cosmétique étant auto-oxydante et prête à l'emploi sans mélange avec un oxydant et comprenant : au moins un précurseur de colorant oxydatif et un agent alcalin pour favoriser la pénétration dudit précurseur de colorant oxydatif au sein de cheveux à colorer, ledit précurseur de colorant oxydatif étant conçu pour être oxydé uniquement par exposition à l'air ambiant pendant au moins une durée prédéterminée pour former, au sein des cheveux à colorer, un colorant de couleur prédéterminée, ladite composition cosmétique incluant en outre au moins une huile végétale, qui inclut de l'huile de graines de *Camellia oleifera* et qui forme un agent filmogène pour favoriser au moins temporairement la rétention dudit colorant au sein des cheveux.

Les objets assignés à l'invention sont également atteints à l'aide d'un procédé de coloration capillaire dans lequel :
- on applique sur des cheveux à colorer une composition cosmétique de coloration capillaire permanente à usage topique, ladite composition cosmétique étant auto-oxydante et prête à l'emploi sans mélange avec un oxydant et comprenant au moins un précurseur de colorant oxydatif, un agent alcalin pour faire pénétrer ledit au moins un précurseur de colorant oxydatif au sein desdits cheveux à colorer, et au moins une huile végétale, qui inclut de l'huile de graines de *Camellia oleifera* et qui forme un agent filmogène pour favoriser au moins temporairement la rétention dudit colorant au sein des cheveux,
- on oxyde ledit au moins un précurseur de colorant oxydatif qui a pénétré au sein desdits cheveux à colorer uniquement par exposition à l'air ambiant pendant au moins une durée prédéterminée pour former, au sein des cheveux à colorer, un colorant de couleur prédéterminée.

Les objets assignés à l'invention sont par ailleurs atteints à l'aide d'un kit de coloration capillaire comprenant une composition cosmétique selon l'invention telle que décrite ci-avant, ainsi qu'un shampoing présentant un pH inférieur à 7, de préférence inférieur à 5.

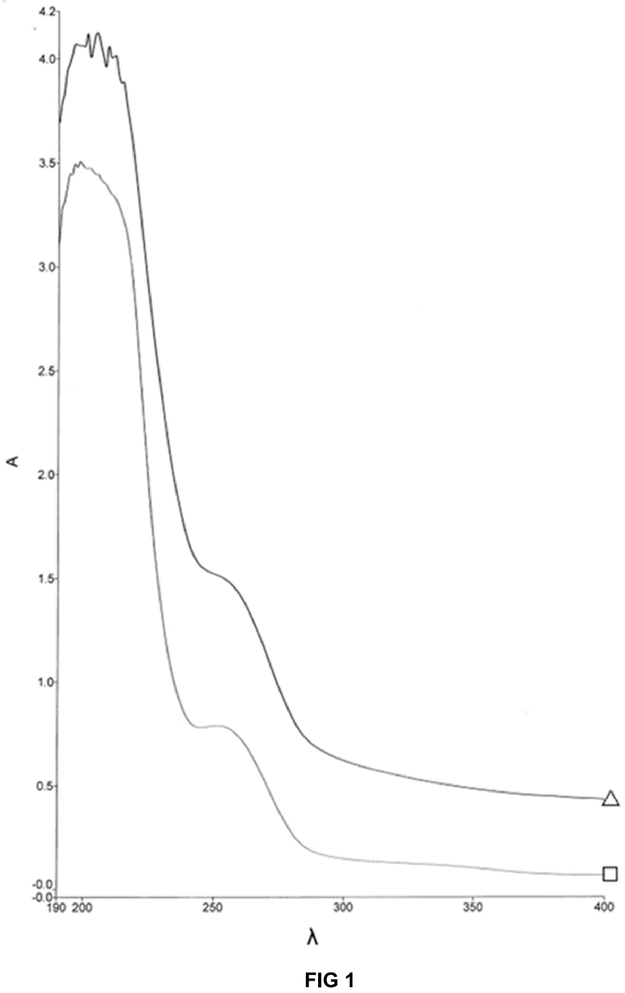

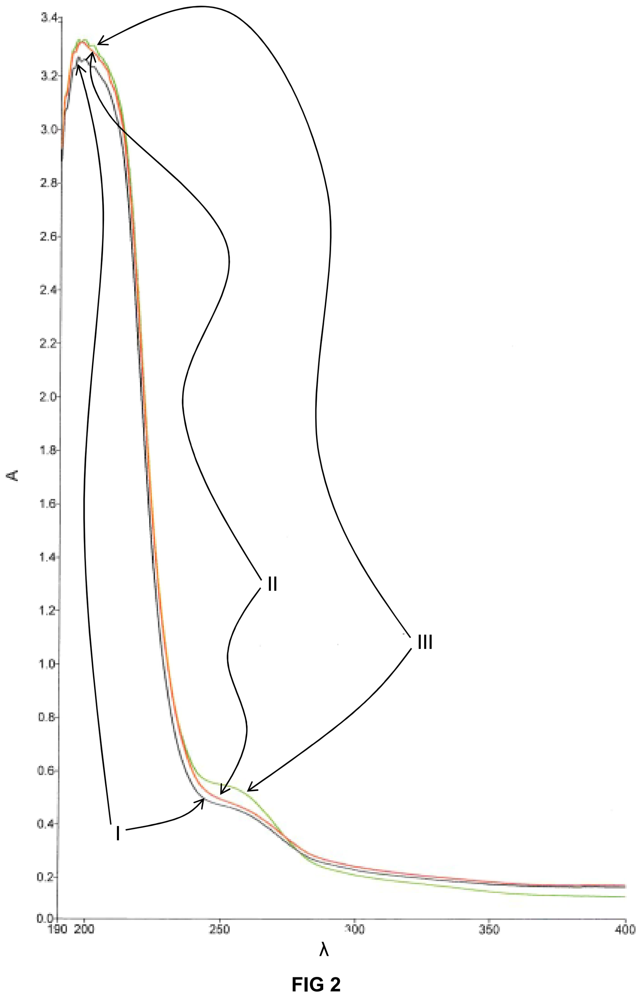

D'autres particularités et avantages de l'invention apparaîtront et ressortiront plus en détail à la lecture de la description faite ci-après, en relation avec les figures 1 et 2 annexées, données uniquement à titre d'exemples illustratifs et non limitatifs. La figure 1 est un graphe d'absorbance (en Unités Arbitraires) en fonction de la longueur d'onde λ (en nm) avec deux courbes correspondant l'une à l'absorbance A d'une eau de rinçage de cheveux colorés au moyen d'une composition selon l'invention et l'autre à l'absorbance A d'une eau de rinçage de cheveux colorés au moyen d'une composition non conforme à l'invention. La figure 2 est un graphe d'absorbance (en Unités Arbitraires) en fonction de la longueur d'onde λ (en nm) avec trois courbes correspondant l'une à l'absorbance A d'une eau de rinçage de cheveux colorés au moyen d'une composition selon l'invention et les deux autres à l'absorbance A d'une eau de rinçage de cheveux colorés au moyen de compositions respectives qui ne sont pas conformes à l'invention.

L'invention concerne une composition cosmétique de coloration capillaire à usage topique, c'est-à-dire qui est destinée à être appliquée directement sur les cheveux, pour colorer ces derniers. La composition cosmétique de coloration capillaire selon l'invention est conçue pour assurer une coloration permanente des cheveux, c'est-à-dire une coloration qui présente une tenue sur une longue durée, ne s'efface pas avec les shampooings et nécessite par exemple simplement un entretien des racines toutes les quatre à six semaines, pour masquer la démarcation créée par la repousse des cheveux.

Avantageusement, la composition cosmétique de coloration capillaire à usage topique selon l'invention est une composition non-éclaircissante, c'est-à-dire qui n'assure pas une décoloration ou une quelconque dégradation de la mélanine des cheveux, ce qui permet en particulier de préserver ces derniers.

Comme cela ressortira plus en détails dans ce qui suit, la composition cosmétique de coloration capillaire selon l'invention est une composition de coloration capillaire oxydante, avec des colorants conçus pour pénétrer les cheveux, dans le cortex de ces derniers.

La composition cosmétique selon l'invention comprend au moins un précurseur de colorant oxydatif et un agent alcalin pour favoriser la pénétration dudit précurseur de colorant oxydatif au sein de cheveux à colorer. Ledit agent alcalin est avantageusement conçu pour ouvrir les cuticules des cheveux, pour permettre ainsi la pénétration du ou des précurseurs de colorant oxydatif dans la fibre capillaire. De préférence, ledit agent alcalin inclut de l'éthanolamine (numéro CAS : 141-43-5). Le recours à de l'éthanolamine en tant qu'agent alcalin s'avère particulièrement avantageux, pour au moins les raisons suivantes :
- l'éthanolamine est peu volatile, notamment par rapport à de l'ammoniaque par exemple, de sorte qu'elle émet moins d'odeurs fortes ;
- du fait de sa volatilité réduite, l'éthanolamine tend à être moins irritante pour le cuir chevelu, la peau et les voies respiratoires ;
- en ouvrant la cuticule de manière progressive et contrôlée, l'éthanolamine limite les risques de dommages aux cheveux (dessèchement, fragilisation), ce qui contribue à préserver la santé et la brillance des cheveux tout en permettant une pénétration suffisante du précurseur de colorant oxydatif dans la fibre capillaire ;
- l'éthanolamine permet d'offrir une excellente stabilité de formulation, facilitant l'obtention d'une composition homogène permettant de conduire à une répartition uniforme de la coloration.

De préférence, le pourcentage massique d'éthanolamine dans la composition cosmétique de coloration est compris entre 5 et 12%. De façon encore plus préférentielle, ledit pourcentage massique est égal à sensiblement 8 %. Cela signifie que l'éthanolamine représente de préférence entre 5 et 12 % de la masse totale de la composition, et de préférence sensiblement 8 % de ladite masse totale de la composition. Le recours à de tels niveaux de pourcentage massique s'avère représenter un excellent compromis entre d'une part la fonction d'ouverture des cuticules et d'autre part la préservation de la qualité et de l'intégrité des fibres capillaires. De tels pourcentages massiques permettent également d'obtenir une bonne stabilité de la formulation, notamment en relation avec les autres ingrédients exposés dans ce qui suit.

Ledit précurseur de colorant oxydatif est conçu pour pénétrer profondément dans le cheveu, grâce à l'action d'ouverture de la cuticule du cheveu exercée par l'agent alcalin. À cette fin, chaque précurseur de colorant oxydatif se présente sous la forme d'une petite molécule, généralement incolore, dont la taille autorise une pénétration dans le cortex du cheveu grâce à l'ouverture de la cuticule.

Une fois à l'intérieur du cheveu, de préférence dans le cortex, le précurseur de colorant oxydatif est conçu pour réagir avec un oxydant pour former de plus grosses molécules colorées, qui restent emprisonnés dans la fibre capillaire, ce qui permet d'obtenir une coloration intense avec une forte rémanence.

Conformément à une caractéristique importante de l'invention, ledit précurseur de colorant oxydatif est conçu pour être oxydé uniquement par exposition à l'air ambiant pendant au moins une durée prédéterminée pour former, au sein des cheveux à colorer, un colorant de couleur prédéterminée. Ladite durée prédéterminée est par exemple comprise entre 30 min et 60 min, de façon plus préférentielle entre 35 min et 50 min, de façon particulièrement encore plus préférentielle entre 35 et 45 min.

En d'autres termes, le précurseur de colorant oxydatif s'oxyde, pour se transformer en pigment coloré, uniquement au contact de l'atmosphère ambiante, en réagissant (oxydation) avec l'oxygène présent dans l'air, qui va agir sur le précurseur de colorant oxydatif pour l'oxyder et ainsi participer à la formation de macro-molécules colorantes. La composition cosmétique de coloration capillaire selon l'invention est donc auto-oxydante, c'est-à-dire qu'elle s'oxyde à l'air, sans avoir besoin d'être mélangée avec un révélateur liquide (type peroxyde d'hydrogène). L'invention permet ainsi de bénéficier d'une composition cosmétique auto-oxydante, prête à l'emploi, sans mélange avec un oxydant. Ceci permet une très grande facilité, fiabilité et sécurité d'utilisation, puisque la coloration est obtenue uniquement en appliquant la composition cosmétique selon l'invention sur les cheveux à colorer, puis en laissant les cheveux ainsi recouverts par la composition être exposés à l'air libre pendant un temps de pose prédéterminé, au terme duquel le précurseur de colorant a pénétré à l'intérieur des cheveux, dans le cortex de ces derniers, pour y être transformé en macro-molécules colorantes, en l'absence de peroxyde d'hydrogène ou toute autre substance oxydante.

Avantageusement, ledit au moins un précurseur de colorant oxydatif inclut un ou plusieurs des précurseurs du groupe suivant :
- sulfate de toluène-2,5-diamine (numéro CAS : 615-50-9) ;
- 4-chlororésorcinol (numéro CAS : 95-88-5) ;
- m-aminophénol (numéro CAS : 591-27-5),
- p-aminophénol (numéro CAS : 123-30-8),
- sulfate de 2-amino-4-hydroxyéthylaminoanisole (numéro CAS : 83763-48-8),
- 2-méthylrésorcinol (numéro CAS : 608-25-3).

De préférence, la composition cosmétique comprend plusieurs précurseurs de colorant oxydatif qui sont tous conçus pour être oxydés uniquement par exposition à l'air ambiant pendant ladite durée prédéterminée, pour développer une coloration souhaitée et/ou réagir avec le ou les autres précurseurs de la composition pour former des macromolécules colorées qui se fixent dans la fibre capillaire. Par exemple, le sulfate de toluène-2,5-diamine réagit, sous l'effet de l'oxydation à l'air libre, avec un ou plusieurs autres précurseurs (comme le 4-chlororésorcinol) pour former des macromolécules présentant une couleur prédéterminée à l'intérieur des cheveux, dans les fibres capillaires, au sein desquelles les précurseurs ont pénétré grâce à l'ouverture des cuticules favorisée par l'agent alcalin.

Avantageusement, la composition cosmétique de coloration capillaire comprend au moins du sulfate de toluène-2,5-diamine et au moins un autre, de préférence au moins deux autres, précurseurs du groupe susvisé. De façon encore plus préférentielle, la composition cosmétique comprend tous les précurseurs de colorant oxydatif dudit groupe susvisé.

Conformément à l'invention, la composition cosmétique de coloration capillaire inclut en outre au moins une huile végétale qui forme un agent filmogène pour favoriser, au moins temporairement, la rétention dudit colorant (obtenu par oxydation à l'air libre dudit au moins un précurseur de colorant oxydatif) au sein des cheveux. La fonction de ladite huile végétale est plus précisément de former un écran protecteur à la surface des cheveux, qui va permettre d'éviter à la coloration de dégorger hors des cheveux lorsque, à l'issue du temps de pose de la composition (correspondant à ladite durée prédéterminée d'exposition à l'air ambiant), la chevelure est rincée et lavée avec un shampooing doux à pH acide pour éliminer toute trace du produit colorant en dehors des cheveux. Le pH acide dudit shampooing doux permet en effet de refermer les écailles de la cuticule ce qui permet aux macromolécules colorantes de rester emprisonnées l'intérieur du cortex ; toutefois, cette action de rinçage et de lavage au shampooing acide peut entraîner un « lessivage » des macromolécules colorées présentes dans les fibres capillaires, affectant ainsi négativement la coloration et en réduisant la rémanence. La présence de l'huile végétale filmogène dans la composition cosmétique permet de remédier à cet inconvénient, en réduisant le phénomène de lessivage évoqué précédemment et en préservant la rémanence de la coloration.

En outre, le processus d'auto-oxydation avec l'oxygène de l'air est progressif et plus lent qu'un processus d'oxydation plus classique en présence de peroxyde d'hydrogène. Ceci est susceptible d'introduire un risque de moindre fixation des molécules colorantes à l'intérieur des fibres capillaires, et donc une moindre rémanence de la coloration. La présence de l'huile végétale formant un agent filmogène permet d'une certaine manière de compenser ce risque et de garantir un excellent niveau de rémanence.

Avantageusement, le pourcentage massique de l'huile végétale dans ladite composition cosmétique est compris entre 0,2 % et 3 %, de préférence entre 0,5 % à 2 %, de façon encore plus préférentielle entre 0,8 et 1,2 %. De tels pourcentages massiques permettent notamment d'assurer un effet significatif de rétention du colorant au sein des cheveux, notamment lors de la phase de rinçage et de shampouinage, tout en permettant de conserver une formulation stable, avec une texture appropriée.

Avantageusement, ladite au moins une huile végétale est riche en acides gras insaturés à longue chaîne, ce qui favorise l'effet filmogène de protection évoqué dans ce qui précède.

Conformément à l'invention, ladite au moins une huile végétale inclut de l'huile de graines de *camellia oleifera* (numéro CAS : 225233-97-6). La présence dans la composition d'huile de graines de *camellia oleifera* formant, au moins en partie voire totalement, une huile végétale filmogène permet de laisser en surface des cheveux un film suffisamment occlusif pour favoriser la rétention du colorant au sein des cheveux lors du rinçage et shampouinage à l'issue du temps de pose. Pour autant, et cela vaut tout particulièrement pour l'huile de graines de *camellia oleifera,* cet effet de rétention n'influe sensiblement pas négativement sur le processus d'auto-oxydation, de sorte qu'un excellent contact entre les précurseurs de colorant oxydatif et l'oxygène de l'air est préservé, grâce au caractère relativement poreux du film formé sur les cheveux par l'huile végétale contenant voire constituée de l'huile de graines de *camellia oleifera.* Ce film souple et perméable empêche ainsi l'extraction intempestive des macromolécules colorantes lors du rinçage et du shampooing final, tout en maintenant les échanges gazeux pendant le temps de pose, ce qui permet au phénomène d'oxydation de se dérouler dans des conditions appropriées et favorables. Cette huile végétale contribue ainsi à former sur les cheveux une couche gainante *« respirante* » qui produit un effet synergique avec les autres ingrédients de la composition, pour les raisons exposées dans ce qui précède.

Dans un mode de réalisation préféré, l'agent filmogène est formé par l'huile de graines de *camellia oleifera,* selon un pourcentage massique dans la composition compris entre 0,5 et 2 %, de préférence entre 0,8 et 1,2 %, plus préférentiellement sensiblement égal à 1 %. Il s'avère en effet que le recours spécifique à l'huile de graines de *camellia oleifera* (numéro CAS : 225233-97-6) permet d'obtenir une excellente rémanence après rinçage et lavage au shampooing au pH acide, comme l'établissent en particulier les essais exposés dans ce qui suit. De plus, l'huile de graines de *camellia oleifera* permet, en plus de sa fonction première d'amélioration de la tenue des pigments colorés au sein des cheveux, de conférer à ces derniers brillance et douceur.

Optionnellement, ladite au moins une huile végétale inclut, en plus de de l'huile de graines de *camellia oleifera,* au moins l'une des huiles suivantes :
- huile de macadamia,
- huile de sésame,
- huile d'argan,
- huile de pépins de courge,
- huile de noyau d'abricot,
- huile d'olive,
- huile d'amande douce,
- huile de ricin,
cette liste n'étant pas limitative.

Les résultats d'essais qui suivent mettent en évidence l'intérêt de recourir à de l'huile de graines de *camellia oleifera* dans la composition selon l'invention.

### Protocole d'essais

### 1. Réalisation de mèches colorées :

1.1. Placer une mèche naturelle dont la masse est d'environ 0.5 g et qui comprend 90% au moins de cheveux blancs sur un papier film.

1.2. A l'aide d'un pinceau, recouvrir la mèche avec la composition de coloration capillaire sur les deux faces de la mèche, en couche épaisse (environ 5 g) pour bien faire prendre le produit. Faire environ 5 aller-retours.

1.3. Laisser poser la mèche ainsi recouverte par la composition de coloration pendant 45 min à température ambiante (sans la couvrir).

### 2. Evaluation de la déperdition en colorant des mèches :

2.1. Prendre la mèche colorée obtenue à l'issue de l'étape 1.3 ci-dessus et la placer sous un robinet de distribution d'eau, qui équipe un évier.

### 2.2. Mouiller complètement la mèche colorée.

2.3. Appliquer une noisette d'un shampoing doux neutralisant de pH égal à 3,7 sur la mèche. Faire mousser en effectuant 5 massages circulaires.

### 2.4. Placer un bécher sous la mèche prête à être rincée.

2.5. Rincer la mèche à l'eau claire en la positionnant entre l'index et le majeur et en effectuant 10 passages de haut en bas sous l'eau du robinet (tout en faisant écouler l'eau de rinçage issu de la mèche dans le bécher placé en dessous).

### 2.6. Répéter 5 fois les étapes 2.3, 2.4 et 2.5.

2.7. Evaluer visuellement l'intensité de la couleur de l'eau de rinçage récupérée.

2.8. Effectuer une analyse instrumentale de l'eau de rinçage au moyen d'un spectrophotomètre (en l'espèce un spectrophotomètre UV/visible modèle Lambda 25 de la société PerkinElmer) pour mesurer l'absorbance de l'eau de rinçage.

### Première série d'essais :

### Essai n°1

Pour l'essai n°1 le protocole d'essais ci-avant est mis en œuvre pour une composition de coloration capillaire selon l'invention conforme à la formulation de la table 1 ci-après.

### Essai n°2

Pour l'essai n°2 le protocole d'essais ci-avant est mis en œuvre dans les mêmes conditions que celles de l'essai n°1, cette fois pour une composition de coloration capillaire qui n'est pas conforme à l'invention, et dont la formulation est strictement identique à celle de la table 1 ci-après, à l'unique différence près que l'huile de graines de *camellia oleifera* est remplacée par de l'eau.

### Résultats et interprétation de la première série d'essais :

Il est visible à l'œil nu que l'eau de rinçage dont la coloration est évaluée à l'étape 2.7 du protocole pour l'essai n°1 est plus claire que l'eau de rinçage dont la coloration est évaluée à l'étape 2.7 du protocole pour l'essai n°2.

Ceci est confirmé par l'analyse spectrophotométrique de l'étape 2.8 du protocole, dont les résultats illustrés par les courbes de la figure 1 annexée montrent une absorbance A plus élevée sur tout le spectre pour l'essai n°2 (courbe marquée par un triangle) que pour l'essai n°1 (courbe marquée par un carré). Ceci traduit le fait que l'eau de rinçage de l'essai n°1 est plus claire, plus transparente, que l'eau de rinçage de l'essai n°2.

Cette différence de transparence s'explique par le fait que l'eau de rinçage de l'essai n°1 est moins chargée en colorant que l'eau de rinçage de l'essai n°2, grâce à l'huile végétale qui a permis de limiter le dégorgement de la coloration hors des cheveux.

### Seconde série d'essais :

### Essai n°1

Pour l'essai n°1 le protocole d'essais ci-avant est mis en œuvre pour une composition de coloration capillaire selon l'invention conforme à la formulation de la table 1 ci-après.

### Essai n°2

Pour l'essai n°2 le protocole d'essais ci-avant est mis en œuvre dans les mêmes conditions que celles de l'essai n°1, cette fois pour une composition de coloration capillaire qui n'est pas conforme à l'invention, et dont la formulation est strictement identique à celle de la table 1 ci-après, à l'unique différence près que l'huile de graines de *camellia oleifera* est remplacée par de l'huile de coco.

### Essai n°3

Pour l'essai n°3 le protocole d'essais ci-avant est mis en œuvre dans les mêmes conditions que celles de l'essai n°1, cette fois pour une composition de coloration capillaire qui n'est pas conforme à l'invention, et dont la formulation est strictement identique à celle de la table 1 ci-après, à l'unique différence près que l'huile de graines de *camellia oleifera* est remplacée par de l'huile d'olive.

### Résultats et interprétation de la seconde série d'essais :

Il est visible à l'œil nu que l'eau de rinçage dont la coloration est évaluée à l'étape 2.7 du protocole pour l'essai n°1 est plus claire que chacune des eaux de rinçage dont la coloration est évaluée à l'étape 2.7 du protocole respectivement pour les essais n°2 et n°3.

Ceci est confirmé par l'analyse spectrophotométrique de l'étape 2.8 du protocole, dont les résultats illustrés par les courbes de la figure 2 annexée montrent, sur la partie du spectre où l'absorbance est significative, une absorbance A plus élevée pour les essais n°2 et n°3 (courbes respectivement référencées Il et III) que pour l'essai n°1 (courbe référencée I). Ceci traduit le fait que l'eau de rinçage de l'essai n°1 est plus claire, plus transparente, que les eaux de rinçage respectives des essais n°2 et n°3.

Cette différence de transparence s'explique par le fait que l'eau de rinçage de l'essai n°1 est moins chargée en colorant que les eaux de rinçage respectives des essais n°2 et n°3, grâce à l'huile de graines de *camellia oleifera* qui a permis de limiter davantage le dégorgement de la coloration hors des cheveux que l'huile de coco et que l'huile d'olive.

On notera par ailleurs que la courbe de l'absorbance A pour l'essai n°1 de la seconde série d'essais, tracée à la figure 2 (courbe référencée 1), n'est pas rigoureusement identique, en valeur et en variation en fonction de la longueur d'onde, à la courbe de l'absorbance A pour l'essai n°1 de la première série d'essais, tracée à la figure 1 (courbe marquée par un carré). Cela s'explique notamment par le fait les deux séries d'essais n'ont pas été faites exactement dans les mêmes conditions et avec le même matériel de mesure : par exemple, les conditions d'homogénéisation appliquées aux eaux de rinçage des essais lors l'analyse spectrophotométrique, bien qu'identiques entre elles pour les essais de la première série et identiques entre elles pour les essais de la seconde série, diffèrent légèrement entre la première série et la seconde série. En revanche, pour une série d'essais donnée parmi la première série et la seconde série, les valeurs d'absorbance mesurées permettent une comparaison relative entre les essais de la série considérée, même si elles ne constituent pas un dosage quantitatif absolu.

La composition selon l'invention est de préférence une composition aqueuse, incluant avantageusement de l'eau, de préférence déminéralisée (numéro CAS : 7732-18-5), selon un pourcentage massique dans la composition qui est par exemple compris entre 55 et 95 %, de façon encore plus préférentielle entre 70 et 90 %, de façon encore plus préférentielle entre 80 et 90%.

Avantageusement, ladite composition cosmétique présente une consistance de gel ou de gelée. Grâce à cette texture très spécifique, l'application est facilitée et permet une répartition particulièrement homogène, idéale pour une application rapide et facile, même sans pinceau. La texture de gel ou de gelée permet de bien enrober les fibres capillaires, sans coulure intempestive, et permet une meilleure pénétration des pigments, tout en étant plus légère et agréable sur le cuir chevelu, avec moins d'effet collant et un rinçage facilité après le temps de pose. Le recours à une consistance de gel ou de gelée s'avère particulièrement bénéfique dans le cas d'une composition cosmétique auto-oxydante comme celle de l'invention. Une telle consistance favorise en effet une pénétration et une répartition homogènes et rapides qui contrebalance le temps de pose rallongé par rapport à une oxydation au peroxyde d'hydrogène. L'oxydation est optimisée grâce au caractère particulièrement homogène et uniforme de l'application obtenue.

De préférence, la composition cosmétique inclut un agent gélifiant pour impartir ladite consistance de gel ou de gelée. Par exemple, le pourcentage massique d'agent gélifiant dans la composition est compris entre 0,5 % et 2 %, de préférence est égal à sensiblement 1,5 %. Avantageusement, ledit agent gélifiant comprend un carbomère (numéro CAS : 9003-01-4). Il est cependant parfaitement envisageable de recourir à un autre agent gélifiant, qu'il soit synthétique ou naturel, comme par exemple une gomme, de l'agar-agar ou bien de l'acide hyaluronique.

Avantageusement, la composition cosmétique comprend en outre un agent chélateur, pour notamment neutraliser les ions métalliques susceptibles d'être présents dans l'eau ou les cheveux (Ca²⁺, Fe³⁺, Cu²⁺) et qui pourraient altérer la stabilité et la performance de la coloration.

L'agent chélateur inclut par exemple du Éthylènediaminetétraacétate tétrasodique (numéro CAS : 64-02-8), à hauteur par exemple de 0,02 à 0,1 % en masse dans la composition.

La table 1 ci-après expose un exemple de composition cosmétique conforme à l'invention, destinée à l'obtention d'une coloration châtain clair de nuance 5.00 dans l'échelle de tons « *International Color Chart »* (ICC).

**Table 1 :**

| **N° CAS** | **Nom INCI Mixte** | **Fonction** | % **INCI massique** |
|---|---|---|---|
| 7732-18-5 | AQUA (WATER) | Solvant | 84,260 |
| 141-43-5 | ETHANOLAMINE | Agent alcalin | 8,000 |
| 615-50-9 | TOLUENE-2,5-DIAMINE SULFATE | Précurseur de colorant oxydatif | 1,950 |
| 9003-01-4 | CARBOMER | Agent gélifiant | 1,500 |
| 225233-97-6 | CAMELLIA OLEIFERA SEED OIL | Agent filmogène | 1,000 |
| 95-88-5 | 4-CHLORORESORCINOL | Précurseur de colorant oxydatif | 0,800 |
| 591-27-5 | m-AMINOPHENOL | Précurseur de colorant oxydatif | 0,600 |
| 123-30-8 | p-AMINOPHENOL | Précurseur de colorant oxydatif | 0,600 |
| 83763-48-8 | 2-AMINO-4-HYDROXYETHYLAMINOANISOLE SULFATE | Précurseur de colorant oxydatif | 0,400 |
| | PARFUM (FRAGRANCE) | Parfum | 0,400 |
| 53694-17-0 | POLYQUATERNIUM-22 | Agent conditionneur | 0,205 |
| 608-25-3 | 2-METHYLRESORCINOL | Précurseur de colorant oxydatif | 0,200 |
| 64-02-8 | TETRASODIUM EDTA | Agent chélateur | 0,085 |
| **TOTAL** | | | **100,000** |

Une composition cosmétique selon l'invention, en particulier celle de l'exemple de la table 1 ci-avant, peut par exemple être fabriquée selon le procédé de fabrication suivant, qui permet d'obtenir une crème à la consistance de gelée lisse, brillante et homogène.

### Préparation d'une composition primaire

On fait chauffer de l'eau déminéralisée à 50°C, et on introduit, sous agitation et sous aspiration tous les précurseurs de colorants oxydatifs ainsi que l'agent chélateur. On agite par exemple pendant 5 à 20 min à 50°C, et on s'assure de l'obtention d'un liquide coloré homogène avec une bonne dispersion des précurseurs de colorant oxydatif.

### Préparation d'une composition secondaire

La composition secondaire est préparée à température ambiante (par exemple à 25°C) de la façon suivante :
- on introduit l'agent gélifiant (carbomère) dans de l'eau déminéralisée en le saupoudrant et l'étalant sur la surface de l'eau ;
- on laisse l'agent gélifiant s'hydrater pendant une durée comprise par exemple entre 10 et 50 min ;
- puis on agite pendant par exemple 5 à 30 min et on chauffe jusqu'à 50°C. On s'assure alors de l'absence de grains et du caractère homogène de cette phase aqueuse.

### Mélange final

On introduit alors dans la composition secondaire ainsi obtenue la composition primaire précédemment obtenue alors que la température de cette dernière est par exemple de 50°C et on agite, par exemple pendant 5 à 30 min, à 50°C pour mélanger ensemble lesdites compositions primaire et secondaire.

On introduit ensuite dans le mélange de l'huile de graines de *camellia oleifera,* et éventuellement un ou plusieurs parfums, et on agite pendant par exemple 5 à 20 min à 50°C. Puis on introduit ensuite l'agent alcalin, et on agite, par exemple pendant 60 à 120 min, à 50°C. Enfin, on introduit par exemple un agent conditionneur, tel que le aqua polyquaternium-22 (numéro CAS : 53694-17-0) et on agite à 50°C pendant par exemple 5 à 30 min.

On obtient alors une composition cosmétique coloration capillaire selon l'invention, qui se présente sous la forme d'une crème lisse, brillante et homogène, avec une consistance de gelée.

L'invention concerne par ailleurs en tant que tel un procédé de coloration capillaire, de préférence au moyen de la composition cosmétique de coloration capillaire à usage topique selon l'invention. Par conséquent, la description qui précède concernant la composition s'applique, *mutatis mutandis,* au procédé de coloration selon l'invention, et inversement la description qui suit relative au procédé est également valable pour la composition selon l'invention.

Selon le procédé de coloration capillaire conforme à l'invention, on applique tout d'abord sur des cheveux à colorer une composition cosmétique de coloration capillaire permanente à usage topique, qui est de préférence celle décrite ci-avant, ladite composition cosmétique étant auto-oxydante et prête à l'emploi sans mélange avec un oxydant et comprenant au moins un précurseur de colorant oxydatif, un agent alcalin pour faire pénétrer ledit au moins un précurseur de colorant oxydatif au sein desdits cheveux à colorer, et au moins une huile végétale, qui inclut de l'huile de graines de *Camellia oleifera* et qui forme un agent filmogène pour favoriser, au moins temporairement, la rétention dudit colorant au sein des cheveux. Ainsi, lors de cette première étape, les cheveux à colorer sont imprégnés d'une solution, formée par ladite composition, contenant l'agent alcalin et un ou plusieurs précurseurs de colorant oxydatif.

L'agent alcalin et l'eau contenus dans la composition appliquée sur les cheveux, de préférence de manière uniforme et homogène, rompent les liaisons ioniques du cheveu, font gonfler la tige capillaire et écartent les écailles. Le ou les précurseur(s) de colorant oxydatif (molécules colorantes) traversent alors la cuticule et se dépose(nt) dans le cortex.

On oxyde ensuite ledit au moins un précurseur de colorant oxydatif qui a pénétré au sein desdits cheveux à colorer uniquement par exposition à l'air ambiant (sans mélange avec un révélateur de type peroxyde d'hydrogène), pendant au moins une durée prédéterminée (temps de pose) pour former, au sein des cheveux à colorer, un colorant de couleur prédéterminée. Lors de cette étape d'oxydation, l'oxygène actif présent dans l'air va agir sur les précurseurs de colorant oxydatif pour les oxyder et ainsi participer à la formation de macro-molécules colorantes. Les pigments de mélanine naturellement présents dans les cheveux ne sont pas impactés par le processus de coloration. Ce processus d'auto-oxydation avec l'oxygène de l'air est progressif, et potentiellement plus lent qu'un processus d'oxydation par mélange avec un révélateur liquide ou semi-liquide comme dans l'art antérieur, de sorte que le temps de pose est adapté et la surface de contact de l'air avec la chevelure imprégnée par la composition doit être optimisée (la chevelure doit être étalée au maximum, et ne pas être recouverte, pour rester à l'air libre).

Avantageusement, une fois ladite durée prédéterminée (temps de pose) écoulée, on rince les cheveux à colorer et on leur applique un shampooing présentant un pH inférieur à 7, de préférence inférieur à 5, par exemple compris entre 3 et 4, pour réduire la perméabilité des cuticules des cheveux à colorer afin d'emprisonner en leur sein au moins ledit colorant. Ainsi, lors de cette étape finale de rinçage et lavage après le temps de pose, la chevelure est soigneusement rincée et un shampooing doux à pH acide (par exemple égal à 3,7) est préférentiellement appliqué pour éliminer toute trace de produits colorants. Le pH acide du shampooing va refermer les écailles de la cuticule et ainsi permettre aux macromolécules colorantes de rester emprisonnées à l'intérieur du cortex. Lors de cette étape finale de rinçage et de lavage, l'action filmogène de l'huile végétale (avantageusement de l'huile de camélia comme précisé auparavant) permet de limiter l'échappement intempestif de macromolécules colorantes hors des fibres capillaires sous l'effet du rinçage et du lavage au shampooing acide. Ceci permet de maintenir une forte rémanence, tout en assurant en outre une fonction de conditionneur capillaire et cutané.

Enfin, l'invention concerne en tant que tel un kit de coloration capillaire qui comprend au moins d'une part une composition cosmétique conforme à l'invention et à la description qui précède, et d'autre part un shampooing présentant un pH inférieur à 7, de préférence inférieur à 5, de façon encore plus préférentielle compris entre 3 et 4. Le kit de coloration capillaire permet ainsi de mettre en œuvre le procédé de coloration capillaire selon l'invention décrit dans ce qui précède. La description qui précède relative à la composition cosmétique de coloration d'une part et au procédé de coloration capillaire d'autre part s'applique donc, *mutatis mutandis,* au kit de coloration capillaire selon l'invention, et réciproquement.

## Revendications

1. Composition cosmétique de coloration capillaire permanente à usage topique, ladite composition cosmétique étant auto-oxydante et prête à l'emploi sans mélange avec un oxydant et comprenant : au moins un précurseur de colorant oxydatif et un agent alcalin pour favoriser la pénétration dudit précurseur de colorant oxydatif au sein de cheveux à colorer, ledit précurseur de colorant oxydatif étant conçu pour être oxydé uniquement par exposition à l'air ambiant pendant au moins une durée prédéterminée pour former, au sein des cheveux à colorer, un colorant de couleur prédéterminée, ladite composition cosmétique incluant en outre au moins une huile végétale, qui inclut de l'huile de graines de *Camellia oleifera* et qui forme un agent filmogène pour favoriser au moins temporairement la rétention dudit colorant au sein des cheveux.

2. Composition cosmétique selon la revendication précédente **caractérisée en ce que** le pourcentage massique de ladite au moins une huile végétale dans ladite composition cosmétique est compris entre 0,2 % et 3 %, de préférence entre 0,5 et 2%, de façon encore plus préférentielle entre 0,8 et 1,2 %.

3. Composition cosmétique selon l'une quelconque des revendications précédentes **caractérisée en ce que** ledit agent filmogène est formé par l'huile de graines de *Camellia oleifera,* selon un pourcentage massique dans la composition compris entre 0,5 et 2 %.

4. Composition cosmétique selon l'une quelconque des revendications précédentes **caractérisée en ce que** ledit agent filmogène est formé par l'huile de graines de *Camellia oleifera,* selon un pourcentage massique dans la composition compris 0,8 et 1,2 %, de préférence sensiblement égal à 1%.

5. Composition cosmétique selon l'une quelconque des revendications précédentes **caractérisée en ce que** ladite au moins une huile végétale inclut en outre au moins l'une des huiles suivantes : huile de macadamia, huile de sésame, huile d'argan, huile de pépins de courge, huile de noyau d'abricot, huile d'olive, huile d'amande douce, huile de ricin.

6. Composition cosmétique selon l'une quelconque des revendications précédentes, **caractérisé en ce que** ledit agent alcalin inclut de l'éthanolamine.

7. Composition cosmétique selon la revendication précédente **caractérisé en ce que** le pourcentage massique d'éthanolamine dans la composition est compris entre 5 et 12%, de préférence est égal à sensiblement 8%.

8. Composition cosmétique selon l'une quelconque des revendications précédentes **caractérisée en ce qu'**elle présente une consistance de gel ou de gelée.

9. Composition cosmétique selon la revendication précédente **caractérisée en ce qu'**elle inclut un agent gélifiant pour impartir ladite consistance de gel ou de gelée.

10. Composition cosmétique selon la revendication précédente **caractérisée en ce que** le pourcentage massique d'agent gélifiant dans la composition est compris entre 0,5 et 2%, de préférence est égal à sensiblement 1,5%, ledit agent gélifiant comprenant par exemple un carbomère.

11. Composition cosmétique selon l'une quelconque des revendications précédentes **caractérisée en ce qu'**elle comprend un agent chélateur, qui inclut par exemple du Éthylènediaminetétraacétate tétrasodique.

12. Composition cosmétique selon l'une quelconque des revendications précédentes **caractérisée en ce que** ledit au moins un précurseur de colorant oxydatif inclut un ou plusieurs des précurseurs du groupe suivant : sulfate de toluène-2,5-diamine, 4-chlororésorcinol, m-aminophénol, p-aminophénol, sulfate de 2-amino-4-hydroxyéthylaminoanisole, 2-méthylrésorcinol.

13. Procédé de coloration capillaire dans lequel :
- on applique sur des cheveux à colorer une composition cosmétique de coloration capillaire permanente à usage topique, ladite composition cosmétique étant auto-oxydante et prête à l'emploi sans mélange avec un oxydant et comprenant au moins un précurseur de colorant oxydatif, un agent alcalin pour faire pénétrer ledit au moins un précurseur de colorant oxydatif au sein desdits cheveux à colorer, et au moins une huile végétale, qui inclut de l'huile de graines de *Camellia oleifera* et qui forme un agent filmogène pour favoriser au moins temporairement la rétention dudit colorant au sein des cheveux,
- on oxyde ledit au moins un précurseur de colorant oxydatif qui a pénétré au sein desdits cheveux à colorer uniquement par exposition à l'air ambiant pendant au moins une durée prédéterminée pour former, au sein des cheveux à colorer, un colorant de couleur prédéterminée.

14. Procédé de coloration capillaire selon la revendication précédente, dans lequel, une fois ladite durée prédéterminée écoulée, on rince lesdits cheveux à colorer et on leur applique un shampoing présentant un pH inférieur à 7, de préférence inférieur à 5, pour réduire la perméabilité des cuticules des cheveux à colorer afin d'emprisonner en leur sein au moins ledit colorant.

15. Kit de coloration capillaire comprenant une composition cosmétique selon l'une quelconque des revendications 1 à 12, ainsi qu'un shampoing présentant un pH inférieur à 7, de préférence inférieur à 5.
